# EUROPÄISCHE PATENTANMELDUNG

(11) **EP 0 982 288 A2**
(43) Veröffentlichungstag der Anmeldung: **01.03.2000**
(21) Anmeldenummer: 99116117.5
(22) Anmeldetag: 18.08.1999
(51) Int. Cl.: C07C 57/07

(54) **Verfahren zur kontinuierlichen Gewinnung von (Meth)acrylsäure**

(30) Priorität: 26.08.1998 DE 19838795
(71) Anmelder: BASF AKTIENGESELLSCHAFT, 67056 Ludwigshafen (DE)
(72) Erfinder: Machhammer, Otto, Dr., 68163 Mannheim (DE); Zehner, Peter, Dr., 67071 Ludwigshafen (DE); Deberdt. Filip, Dr., 2812 Muinzen (BE)
(74) Vertreter: Isenbruck, Günter, Dr.

(57) **Zusammenfassung**

Es wird ein Verfahren zur kontinuierlichen Gewinnung von (Meth)acrylsäure aus den Reaktionsgasen einer katalytischen Gasphasenoxidation durch (I) Absorption in einem hochsiedenden Lösungsmittel, (II) Abtrennung der (Meth)acrylsäure aus dem Gemisch mit dem Lösungsmittel und gegebenenfalls weitere Reinigung der abgetrennten (Meth)acrylsäure, (III) Reinigung des Lösungsmittels und (IV) Rückführung des gereinigten Lösungsmittels in die Absorptionsstufe (I) vorgeschlagen, wobei die Temperatur in jeder Verfahrensstufe 155°C, insbesondere 140°C, bevorzugt 120° nicht übersteigt.

## Beschreibung

Die Erfindung betrifft ein Verfahren zur kontinuierlichen Gewinnung von (Meth)acrylsäure durch Absorption von (Meth)acrylsäure aus den Reaktionsgasen einer katalytischen Gasphasenoxidation. Der Begriff (Meth)acrylsäure steht im folgenden für die Substanzen Acrylsäure und/oder Methacrylsäure.

(Meth)acrylsäure wird überwiegend durch katalytische Gasphasenoxidation geeigneter Ausgangsstoffe, insbesondere von Propen und/oder Acrolein im Falle der Acrylsäure bzw. von Isobuten und/oder Methacrolein im Falle der Methacrylsäure, hergestellt.

Zur Abtrennung der (Meth)acrylsäure aus den Reaktionsgasen der katalytischen Gasphasenoxidation sind eine Reihe von Möglichkeiten bekannt, darunter auch die Abtrennung durch Absorption in ein Lösungsmittel.

Aus DE-B 21 36 396 ist bekannt, die Acrylsäure aus den bei der katalytischen Oxidation von Propen bzw. Acrolein erhaltenen Reaktionsgasen durch Gegenstromabsorption mit einem Gemisch aus 75 Gew.-% Diphenylether und 25 Gew.-% Diphenyl abzutrennen. Weiterhin ist aus DE-A 24 49 780 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten, insbesondere bei der destillativen Reinigung der (Meth)acrylsäure, der Anfall von Feststoffen in den Apparaten, der die Anlagenverfügbarkeit reduziert. Gemäß DE-A 43 08 087 kann im Fall der Acrylsäure dieser Feststoffanfall reduziert werden, indem man dem relativ unpolaren Lösungsmittelgemisch aus Diphenylether und Diphenyl ein polares Lösungsmittel wie Dimethylphthalat in einer Menge von 0,1 bis 25 Gew.-% zufügt; dadurch erhöht sich das Aufnahmevermögen des Lösungsmittelgemisches für die schmutzbildenden Stoffe. Mit steigender Polarität nimmt das Lösungsmittel jedoch zunehmende Mengen an Wasser mit auf; außerdem führt dies zu erhöhten Lösungsmittelverlusten über das Sauerwasser.

In Gegenwart von Lösungsmitteln bildet die Polyacrylsäure im Bereich höherer Temperaturen, wie sie bei der Gewinnung von (Meth)acrylsäure nach dem gattungsgemäßen Verfahren, insbesondere am untersten Sammelboden der Absorptionskolonne, im Abtriebs- und Sumpfteil der Destillationskolonne sowie in den Wärmetauschern auftreten, einen an der Oberfläche der Apparate fest haftenden Schmutz, der nur mit Laugen gelöst werden kann. Analysen haben gezeigt, daß der Schmutz aus einer Mischung aus ca. 10 bis 50 Gew.-% Poly(meth)acrylsäure, Rest Lösungsmittel, besteht.

Es ist daher Aufgabe der Erfindung, die Verschmutzungsanfälligkeit in sämtlichen Apparaten, insbesondere den Anfall von nur lauge-löslichen Verschmutzungen weitgehend zu vermeiden, und somit die Anlagenverfügbarkeit und die Wirtschaftlichkeit des Verfahrens zur Gewinnung der (Meth)acrylsäure zu verbessern.

Die Erfindung geht aus von einem Verfahren zur kontinuierlichen Gewinnung von (Meth)acrylsäure aus den Reaktionsgasen einer katalytischen Gasphasenoxidation durch
(I) Absorption der (Meth)acrylsäure in ein hochsiedendes Lösungsmittel,
(II) Abtrennung der (Meth)acrylsäure aus dem Gemisch mit dem Lösungsmittel und gegebenenfalls weitere Reinigung der abgetrennten (Meth)acrylsäure
(III) Reinigung des Lösungsmittels und
(IV) Rückführung des gereinigten Lösungsmittels in die Absorptionsstufe (I).

Das Verfahren ist dann dadurch gekennzeichnet, daß die Temperatur in jeder Verfahrensstufe 155°C, insbesondere 140°C, besonders bevorzugt 120°C, nicht übersteigt.

Es wurde überraschenderweise gefunden, daß die Verschmutzungsanfälligkeit von Apparaten, in denen ein hochsiedendes Lösungsmittel sowie (Meth)acrylsäure eingesetzt werden, im wesentlichen von einem Verfahrensparameter, und zwar von der Temperatur in den Apparaten bestimmt wird. Die Verschmutzung kann weitgehend vermieden werden, wenn sichergestellt ist, daß die Temperatur einen bestimmten kritischen Wert nicht übersteigt.

Dagegen nimmt das Fouling in den Apparaten nicht, wie erwartet, mit steigender (Meth)acrylsäure-Konzentration zu, die Verschmutzung ist im Gegenteil von der (Meth)acrylsäure-Konzentration weitgehend unabhängig.

Die weitgehende Vermeidung von Verschmutzung durch die erfindungsgemäße Temperaturbegrenzung hat weitreichende wirtschaftliche Konsequenzen: insbesondere können in den Apparaten Elemente eingesetzt werden, wie Füllkörper oder strukturierte Packungen, die eine größere hydrodynamische Belastbarkeit, jedoch auch eine höhere Verschmutzungsanfälligkeit gegenüber beispielsweise Dual-Flow- oder Ventilböden aufweisen, die nach dem bekannten Verfahren zur Abtrennung von (Meth)acrylsäure aus Gemischen in einem hochsiedenden Lösungsmittel wegen ihrer geringeren Verschmutzungsanfälligkeit eingesetzt werden. Neuanlagen können somit bei gleicher Produktionsmenge mit kleineren Trennapparaten, insbesondere Kolonnen, dimensioniert werden, bzw. wird bei bestehenden Anlagen die Produktionsmenge nach dem erfindungsgemäßen Verfahren erhöht.

Als hochsiedend werden vorliegend Lösungsmittel bezeichnet, deren Siedepunkt höher ist als der Siedepunkt des jeweils angestrebten Hauptprodukts (ca. 141°C für Acrylsäure bzw. ca. 161°C für Methacrylsäure, jeweils bei Normaldruck).

Ausgangsgemische für das vorliegende Verfahren sind die Reaktionsgase aus der katalytischen Gasphasenoxidation von C₃-Alkanen, -Alkenen, -Alkanolen und/oder - Alkanalen oder Vorstufen davon zu Acrylsäure bzw. von C₄-Alkanen, C₃-Alkanen, - Alkenen, -Alkanolen und/oder -Alkanalen oder Vorstufen davon zu Methacrylsäure. Das Verfahren wird im folgenden für Acrylsäure beschrieben, es gilt jedoch analog auch für Methacrylsäure.

Besonders vorteilhaft ist die katalytische Gasphasenoxidation von Propen und/oder Acrolein zu Acrylsäure mit Luft oder molekularem Sauerstoff nach bekannten Verfahren, insbesondere wie sie in den oben genannten Druckschriften beschrieben sind. Vorzugsweise wird hierbei bei Temperaturen zwischen 200 und 450 °C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch katalytische Oxidehydrierung, wie in US-5 510 558 beschrieben oder durch homogene Oxidehydrierung, wie z. B. in CN-A-1 105 352 beschrieben; oder durch katalytische Dehydrierung, entsprechend dem Beispiel der EP-A-0 253 409. Bei Einsatz eines Propen-/Propan-Gemischs wirkt Propan als Verdünnungsgas. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die o. g. mit Sauerstoff oder Luft oder einem Gemisch aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z.B. Kreisgas (siehe unten), Luftstickstoff, einen oder mehrere gesättigte C₁- bis C₆-Kohlenwasserstoffe, insbesondere Methan und/oder Propan und/oder Wasserdampf enthält, kann nur einen kleinen Teil der Reaktionswärme aufnehmen. Obwohl die Art der verwendeten Reaktoren an sich keiner Beschränkung unterliegt, werden meist salzbadgekühlte Rohrbündelwärmetauscher verwendet, die mit dem Oxidationskatalysator gefüllt sind, da bei diesen die bei der Reaktion freiwerdende Wärme sehr gut durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Säuren und Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% der Summe aus Maleinsäure und Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 95 Gew.-% Methan und/oder Propan, daneben 1 bis 30 Gew.-% Wasserdampf, 0,05 bis 15 Gew.-% Kohlenoxide und 0 bis 95 Gew.-% Stickstoff, jeweils bezogen auf 100 Gew.-% Reaktionsgas, enthalten.

Die Verfahrensstufen zur Abtrennung der Acrylsäure aus dem Reaktionsgemisch werden im folgenden beschrieben:

### Stufe I

In Stufe I werden die Acrylsäure und ein Teil der Nebenkomponenten aus dem Reaktionsgas durch Absorption in einem hochsiedenden Lösungsmittel absorbiert. Vorzugsweise liegt der Siedepunkt des hochsiedenden Lösungsmittels wenigstens 20°C, insbesondere 50°C, stärker bevorzugt 70°C über dem Siedepunkt der Acrylsäure bzw. Methacrylsäure. Bevorzugte Lösungsmittel, wobei in vorliegender Anmeldung der Begriff Lösungsmittel auch Lösungsmittelgemische umfaßt, haben Siedepunkte (bei Normaldruck) von 180 bis 400°C, insbesondere von 220 bis 360°C. Geeignete Lösungsmittel sind hochsiedende, extrem hydrophobe Lösungsmittel, die keine nach außen wirkende polare Gruppe enthalten, wie z.B. aliphatische oder aromatische Kohlenwasserstoffe, z.B. Mittelölfraktionen aus der Paraffindestillation, oder Äther mit sperrigen Gruppen am O-Atom, oder Gemische davon, wobei diesen vorteilhafterweise ein polares Lösungsmittel, wie das in DE-A-43 08 087 offenbarte 1,2-Dimethylphthalat zugesetzt wird. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkane, z.B. 4-Methyl-4'-benzyl-diphenylmethan und dessen Isomere 2-Methyl-2'-benzyl-diphenylmethan, 2-Methyl-4'-benzyl-diphenylinethan und 4-Methyl-2'-benzyl-diphenylmethan und Gemische solcher Isomerer.

Ein besonders bevorzugtes Lösungsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, bezogen auf 100 Gew.-% Diphenyl und Diphenylether, beispielsweise das im Handel erhälfliche Diphyl®. Vorzugsweise enthält dieses Lösungsmittelgemisch weiterhin ein polares Lösungmittel wie Dimethylphthalat in einer Menge von 0,1 bis 25 Gew.-%, bezogen auf das gesamte Lösungsmittelgemisch. Dadurch reduziert sich die Verschmutzungsanfälligkeit der Anlagen.

Vorliegend bezeichnen die Begriffe Hoch- oder Schwersieder, Mittelsieder und Leichtsieder sowie entsprechend adjektivisch gebrauchte Begriffe Verbindungen, die einen höheren Siedepunkt als die Acrylsäure besitzen (Hochsieder) bzw. solche, die in etwa den gleichen Siedepunkt wie Acrylsäure besitzen (Mittelsieder) bzw. solche, die einen niedrigeren Siedepunkt als Acrylsäure besitzen (Leichtsieder).

Vorteilhafterweise wird das heiße Reaktionsgas durch Teilverdampfen des Lösungsmittels in einem Direktkondensator oder Quenchapparat, vor der Absorption abgekühlt. Hierfür eignen sich insbesondere Venturiwäscher, Blasensäulen oder Sprühkondensatoren. Dabei kondensieren die schwersiedenden Nebenkomponenten des Reaktionsgases in das nicht verdampfte Lösungsmittel. Außerdem ist die Teilverdampfung des Lösungsmittels ein Reinigungsschritt für das Lösungsmittel. In einer bevorzugten Ausführungsform der Erfindung wird ein Teilstrom des nicht verdampften Lösungsmittels, vorzugsweise 1 bis 10 % des der Absorptionskolonne zugeführten Massenstroms, abgezogen und einer Lösungsmittelreinigung unterworfen. Hierbei wird das Lösungsmittel überdestilliert und zurück bleiben die schwersiedenden Nebenkomponenten, die - bei Bedarf weiter eingedickt - entsorgt, z.B. verbrannt, werden können. Diese Lösungsmitteldestillation dient der Vermeidung einer zu hohen Konzentration an Schwersiedern im Lösungsmittelstrom. Das überdestillierte Lösungsmittel wird vorzugsweise dem beladenen Lösungsmittelstrom aus der Absorptionskolonne zugeführt.

Die Absorption erfolgt in einer Gegenstromabsorptionskolonne, die grundsätzlich mit jeder Art von Kolonneneinbauten, vorzugsweise mit Füllkörpern oder strukturierten Packungen bestückt ist, und die von oben mit Lösungsmittel beaufschlagt wird. Das gasförmige Reaktionsprodukt und gegebenenfalls verdampftes Lösungsmittel aus dem Quenchapparat werden von unten in die Kolonne eingeleitet und anschließend auf Absorptionstemperatur abgekühlt. Die Abkühlung erfolgt vorteilhafterweise durch Kühlkreise, d.h. erwärmtes Lösungsmittel wird aus der Kolonne abgezogen, in Wärmetauschern abgekühlt und wieder an einer Stelle oberhalb der Abzugsstelle der Kolonne zugeführt. Nach der Absorption befinden sich alle Schwersieder der größte Teil der Acrylsäure sowie einen Teil der Leichtsieder im Lösungsmittel.

Das verbleibende, nicht absorbierte Reaktionsgas wird weiter abgekühlt, um den kondensierbaren Teil der leichtsiedenden Nebenkomponenten insbesondere Wasser, Formaldehyd und Essigsäure, durch Kondensation davon, abzutrennen. Dieses Kondensat wird im folgenden Sauerwasser genannt. Der verbleibende Gasstrom besteht überwiegend aus Stickstoff, Kohlenoxiden und nicht umgesetzten Edukten. Vorzugsweise wird dieser teilweise wieder als Verdünnungsgas, im folgenden Kreisgas genannt, den Reaktionsstufen zugeführt. Der Luftstickstoff und ein Teil der nichtkondensierten Nebenkomponenten werden als Abgas ausgeschleust und vorzugsweise verbrannt.

### Stufe II

In Verfahrensstufe II wird die Acrylsäure zusammen mit den mittelsiedenden Komponenten sowie dem letzten Rest an leichtsiedenden Nebenkomponenten vom Lösungsmittel abgetrennt.

In bevorzugter Weise erfolgt die Abtrennung der Acrylsäure aus dem Gemisch mit dem Lösungsmittel durch Verdampfung. Die Verdampfung wird bei einem Druck zwischen 10 und 200 mbar und entsprechenden Verdampfungstemperaturen von 60° bis 130°C, insbesondere bei einem Druck von 60 bis 100 mbar und Temperaturen von 90° bis 110°C, durchgeführt. Als Ergebnis der Verdampfung entsteht ein Brüdenstrom sowie ein Flüssigstrom. Der Brüdenstrom enthält den größten Teil der Acrylsäure, d.h. Acrylsäurekonzentrationen von ca. 70 bis 95 %, insbesondere von ca. 80 bis 90%. Der Flüssigstrom aus dem Verdampfer enthält vorwiegend das Lösungsmittel sowie Acrylsäure in einer Konzentration von ca. 5 bis 15 Gew.-%. Dieser Flüssigstrom muß anschließend gereinigt werden, vorzugsweise durch Strippen. Die Reinigung des Lösungsmittels wird nachfolgend als Verfahrensstufe (III) beschrieben.

Die Acrylsäure wird daraus in weiteren Verfahrensschritten, beispielsweise destillativ oder über Kristallisation gereinigt.

Gemäß einer weiteren Ausführungsform erfolgt die Abtrennung der Acrylsäure mittels Destillation, wobei grundsätzlich jede Destillationskolonne verwendet werden kann. Vorteilhafterweise wird hierzu eine Kolonne mit Dual-flow-Böden verwendet. Im Auftriebsteil der Kolonne wird die Acrylsäure vom Lösungsmittel und den mittelsiedenden Nebenkomponenten, wie Maleinsäureanhydrid, frei destilliert. Um den Leichtsiederanteil in der Acrylsäure zu reduzieren, wird vorteilhafterweise der Auftriebsteil der Kolonne verlängert und die Acrylsäure als Seitenabzug aus der Kolonne abgezogen. Diese Acrylsäure wird Roh-Acrylsäure genannt.

Am Kopf der Kolonne wird dann nach einer Partialkondensation ein an Leichtsiedern reicher Strom abgezogen. Da dieser Strom aber noch Acrylsäure enthält, wird er vorteilhafterweise nicht verworfen, sondern der Absorptionsstufe (I) rückgeführt.

Aus dem Sumpf der Kolonne wird ein Strom, der vorwiegend Lösungsmittel enthält, abgezogen und der Stufe III zugeführt.

In einer bevorzugten Ausführungsform der Erfindung wird das Sauerwasser, das noch Acrylsäure gelöst enthalten kann, mit einem Meinen Teilstrom des nahezu Acrylsäure-freien Lösungsmittels (aus Stufe III) extraktiv behandelt. Der wäßrige Strom aus der Sauerwasserextraktion kann, was insbesondere bei Vorhandensein von Umweltschutzauflagen erforderlich sein kann, eingeengt werden.

Die in Stufe II erhaltene Roh-Acrylsäure enthält, jeweils bezogen auf die Roh-Acrylsäure, vorzugsweise 98 bis 99,8 Gew.-%, insbesondere 98,5 bis 99,5 Gew.-%, Acrylsäure und 0,2 bis 2 Gew.-%, insbesondere 0,5 bis 1,5 Gew.-% Verunreinigungen, wie z.B. Essigsäure, Aldehyde und Maleinsäureanhydrid. Diese Acrylsäure kann, sofern die Anforderungen an ihre Reinheit nicht sehr hoch sind, gegebenenfalls bereits zur Veresterung verwendet werden.

Im Falle der destillativen Durchführung der Verfahrensstufe II (Abtrennung der Acrylsäure aus dem Gemisch mit dem Lösungsmittel und gegebenenfalls weitere Reinigung der abgetrennten Acrylsäure) kann die Bedingung gemäß der Erfindung, daß die Temperatur in jeder Verfahrensstufe 155°C, insbesondere 140°C, besonders bevorzugt 120°C nicht übersteigt, im wesentlichen durch zwei Maßnahmen realisiert werden:
zum einen durch Zulassung einer erhöhten Acrylsäurekonzentration im Sumpf der Destillationskolonne und zum anderen durch Erniedrigung des Sumpfdrucks.

Bei dem Verfahren nach dem Stand der Technik wird die Destillation bei einem Kopfdruck von etwa 100 mbar betrieben, was durch den Druckverlust über die Kolonnenböden einem Sumpfdruck von ca. 250 mbar und damit einer Sumpftemperatur von ca. 195°C und einer Acrylsäurekonzentration von ca. 0,5 Gew.-% im Sumpfprodukt entspricht.

Wird eine Acrylsäurekonzentration im Bereich von 5 bis 15 Gew.-%, insbesondere von 8 bis 12 Gew.-% im Sumpfprodukt zugelassen, so wird es möglich, bei gleichem Sumpfdruck von ca. 250 mbar eine Sumpftemperatur < 155°C einzuhalten, und damit den Foulinggrad drastisch zu reduzieren.

Dieses Sumpfprodukt kann wegen des hohen Acrylsäuregehaltes von 8 bis 12 Gew.-% nicht ohne weiteres zur Absorptionskolonne (Stufe I) rezirkuliert werden sondern muß vorher gereinigt werden, vorzugsweise durch Strippen. Die Reinigung des Lösungsmittels wird im folgenden als Verfahrensstufe III beschrieben.

Bei einer gegebenen Zusammensetzung des Sumpfprodukts kann die erfindungsgemäße Temperaturbegrenzung auf 155°C, insbesondere 140°C, besonders bevorzugt 120°C durch Absenken des Sumpfdrucks erreicht werden.

Der Sumpfdruck kann im wesentlichen durch zwei Maßnahmen erniedrigt werden: durch Erniedrigung des Kopfdrucks der Destillationskolonne und/oder durch Reduzieren des Druckverlustes in der Kolonne, beispielsweise durch Einsatz geeigneter Kolonneneinbauten, wie strukturierte Packungen. Durch Absenkung des Kopfdrucks auf ca. 10 mbar kann ein Sumpfdruck von ca. 170 bis 200 mbar eingestellt werden. Werden zusätzlich spezielle Kolonneneinbauten, beispielsweise strukturierte Packungen, verwendet, so läßt sich der Sumpfdruck bis auf ca. 30 mbar absenken.

### Stufe III und IV

Vor der Rückführung in die Absorptionsstufe I muß der Lösungsmittelstrom weitgehend von Acrylsäure abgereinigt werden, um erneut Acrylsäure aus dem Reaktionsgas der Gasphasenoxidation aufnehmen zu können. Die Acrylsäurekonzentration im Lösungsmittelstrom soll dabei 1 Gew.-%, bevorzugt 0,5 Gew.-% nicht übersteigen.

Der in Stufe (II) anfallende Lösungsmittelstrom kann noch größere Mengen an Acrylsäure, bis etwa 15 Gew.-% enthalten. Die Abreicherung des Lösungsmittels von Acrylsäure erfolgt in bevorzugter Weise durch Strippen mit einem Inertgas oder einem inerten Gasgemisch, bevorzugt mit einem Teilstrom des Kreisgases oder im Fall, daß Propan Verdünnungsmittel ist, mit Propan. Das Strippen erfolgt bei Drücken von etwa 1,1 bis 2,0 bar, bevorzugt bei Drücken von 1,3 bis 1,6 bar sowie bei Temperaturen von ca. 80 bis 120°C, bevorzugt von 110 bis 120°C. Beim Strippen wird der zu reinigende Lösungsmittelstrom am Kopf einer Strippkolonne aufgegeben; er fließt über die Einbauten in Richtung Sumpf. Im Gegenstrom wird in den Sumpf der Strippkolonne das Strippgas eingeleitet. Während das Strippgas in Richtung Kolonnenkopf strömt, nimmt es Acrylsäure aus dem flüssigen Lösungsmittelstrom auf, so daß aus dem Sumpf der Strippkolonne ein gereinigter Lösungsmittelstrom abgezogen werden kann, der eine Acrylsäurekonzentration von maximal 1 Gew.-%, bevorzugt von maximal 0,5 Gew.-% enthält. Dieses weitgehend acrylsäurefreie Lösungsmittel kann anschließend wieder zur Absorptionsstufe (I) rezirkuliert werden.

Das mit Acrylsäure beladene Strippkreisgas wird zweckmäßigerweise in die Stufe, in der die Teilverdampfung des Lösungsmittels erfolgt, oder in die Absorptionskolonne rezirkuliert.

Die Erfindung wird im folgenden anhand einer Figur sowie anhand von Ausführungsbeispielen näher erläutert.

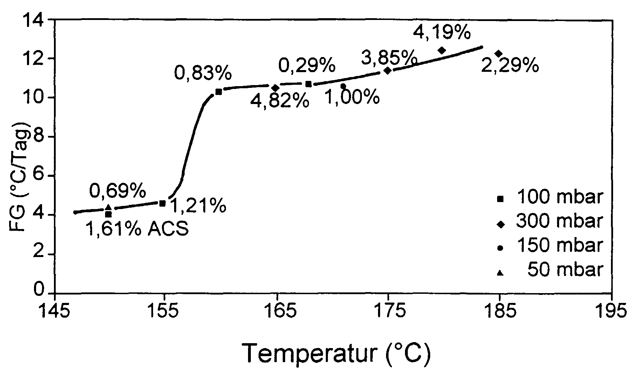

- Fig. 1:: zeigt den in °C/Tag ausgedrückten Foulinggrad (FG) in einem am Sumpf der Destillationskolonne angeschlossenen Wärmetauscher in Abhängigkeit von der Sumpftemperatur.

Der Foulinggrad ist ein Maß für die Vermutzung der Kolonne. Er gibt an, um wieviel °C die Vorlauftemperatur des Wärmeträgermediums im Sumpfverdampfer pro Tag ansteigen muß, um eine gleichbleibende Menge an Sumpfprodukt zu verdampfen. Wie die Figur zeigt, haue die Acrylsäurekonzentration im Sumpfprodukt, die zwischen 0,29 Gew.-% und 4,82 Gew.-% variiert wurde, auf den Foulinggrad keinen Einfluß. Überraschenderweise wurde gefunden, daß der Foulinggrad maßgeblich von der Sumpftemperatur bestimmt wird. Der Foulinggrad ist relativ gering und steigt unterhalb 155°C nur langsam mit der Temperatur; er zeigt einen sprunghaften Anstieg bei etwa 160°C und steigt mit weiterer Temperaturerhöhung nur mäßig an.

### Beispiele

In einer Glaskolonne mit einem Durchmesser von 30 mm und einer Höhe von 3 m, die mit 30 Dual-Flow-Böden aus V4A-Edelstahl mit Teflondichtungen bestückt war, wurde ein flüssiges Ausgangsgemisch mit einer Zulauftemperatur von 50°C und einer Zusammensetzung von ca. 15 Gew.-% Acrylsäure, 0,5 Gew.-% Essigsäure, 0,1 Gew.-% Wasser und Rest ein Diphenyl/Dimetyhlphthalat-Gemisch von 80:20 Gewichtsteilen 8 Böden über dem Sumpf zugegeben. Dem Sumpf war ein Umlauf-Entspannungsverdampfer, dem Kopf der Kolonne ein Quench-Kondensator angeschlossen. Das Produkt wurde über einen Seitenabzug, 5 Böden unter dem Kolonnenkopf, abgeleitet.

Für das Vergleichsbeispiel wurden Betriebsbedingungen eingestellt, die dem Stand der Technik am nächsten kommen:
p_{Kopf} = 390 mbar, T_{Kopf} = 105°C,
p_{Sumpf} = 300 mbar, T_{Sumpf} = 185°C,
c_{Acrylsäure im Sumpf} = 2,29 Gew.-%

Im Umlauf-Entspannungsverdampfer wurde ein Foulinggrad von 12°C pro Tag gemessen.

Die folgenden Verfahrensbeispiele beziehen sich auf dieselbe Versuchsanordnung, wobei die Betriebsparameter jeweils entsprechend den nachfolgenden Angaben geändert wurden:

### Beispiel 1:

p_{Kopf} = 45 mbar, T_{Kopf} = 61°C,
p_{Sumpf} = 50 mbar, T_{Sumpf} = 150°C,
c_{Acrylsäure im Sumpf} = 1,61 Gew.-%.

Der Foulinggrad im Umlauf-Entspannungsverdampfer betrug 4°C pro Tag.

### Beispiel 2:

Es wurden die folgenden Betriebsparameter eingestellt:
p_{Kopf} = 90 mbar, T_{Kopf} = 78°C,
p_{Sumpf} = 100 mbar, T_{Sumpf} = 155°C,
c_{Acrylsäure im Sumpf} = 1,21 Gew.-%.

Der Foulinggrad im Umlauf-Entspannungsverdampfer betrug 4,5°C pro Tag.

### Beispiel 3:

Bei den Betriebsbedingungen:
p_{Kopf} = 90 mbar, T_{Kopf} = 78°C,
p_{Sumpf} = 100 mbar, T_{Sumpf} = 157,5°C,
c_{Acrylsäure im Sumpf} = 0,83 Gew.-%.
betrug der Foulinggrad im Umlauf-Entspannungsverdampfer 10,2°C pro Tag.

### Beispiel 4:

Bei den Betriebsbedingungen
p_{Kopf} = 390 mbar, T_{Kopf} = 105°C,
p_{Sumpf} = 300 mbar, T_{Sumpf} = 165°C,
c_{Acrylsäure im Sumpf} = 4,82 Gew.-%.
betrug der Foulinggrad im Umlauf-Entspannungsverdampfer ca. 12,5°C pro Tag.

## Patentansprüche

1. Verfahren zur kontinuierlichen Gewinnung von (Meth)acrylsäure aus den (Meth)acrylsäure enthaltenden Reaktionsgasen einer katalytischen Gasphasenoxidation durch
(I) Absorption der (Meth)acrylsäure in ein hochsiedendes organisches Lösungsmittel,
(II) Abtrennung der (Meth)acrylsäure aus ihrem Gemisch mit dem Lösungsmittel aus (I) und gegebenenfalls weitere Reinigung der abgetrennten (Meth)acrylsäure
(III) Reinigung des Lösungsmittels und
(IV) Rückführung des gereinigten Lösungsmittels in die Absorptionsstufe (I),
dadurch gekennzeichnet, daß die Temperatur in jeder Verfahrensstufe 155°C, insbesondere 140°C, besonders bevorzugt 120°C, nicht übersteigt.

2. Verfahren nach Anspruch 1, dadurch gekennzeichnet, daß das hochsiedende Lösungsmittel Diphenyl und Diphenylether, bevorzugt in der azeotropen Zusammensetzung, enthält.

3. Verfahren nach Anspruch 2, dadurch gekennzeichnet, daß das hochsiedende Lösungsmittel 0,1 bis 25 Gew.-% Dimethylphthalat enthält.

4. Verfahren nach einem der Ansprüche 1 bis 3, dadurch gekennzeichnet, daß die (Meth)acrylsäure in Stufe II durch Verdampfung abgetrennt wird und anschließend durch Destillation oder Kristallisation weiter gereinigt wird.

5. Verfahren nach Anspruch 4, dadurch gekennzeichnet, daß die Verdampfung bei einem Druck von 10 bis 200 mbar und einer Temperatur von 60° bis 130°C, insbesondere bei einem Druck von 60 bis 100 mbar und einer Temperatur von 90°C bis 110°C, durchgeführt wird.

6. Verfahren nach einem der Ansprüche 1 bis 3, wonach die (Meth)acrylsäure durch Destillation aus dem Gemisch mit dem hochsiedenden Lösungsmittel abgetrennt wird, dadurch gekennzeichnet, daß im Sumpfprodukt der Destillation eine (Meth)acrylsäurekonzentration von 5 bis 15 Gew.-%, insbesondere von 8 bis 12 Gew.-%, eingestellt wird.

7. Verfahren nach einem der Ansprüche 1 bis 6, dadurch gekennzeichnet, daß das Lösungsmittel durch Strippen mit einem Inertgas, insbesondere mit einem Teilstrom des Kreisgases, gereinigt wird.
